# EUROPEAN PATENT APPLICATION

(11) **EP 4 289 837 A1**
(43) Date of publication of application: **13.12.2023**
(21) Application number: 22749540.5
(22) Date of filing: 25.01.2022
(51) Int. Cl.: C07D 405/12, C08G 59/00

(54) **NOVEL COMPOUND, AND CURABLE RESIN COMPOSITION CONTAINING SAID COMPOUND**

(30) Priority: 03.02.2021 JP 2021016043
(71) Applicant: ADEKA CORPORATION, Arakawa-ku Tokyo 116-8554 (JP)
(72) Inventor: TAMASO, Ken-ichi, Kuki-shi, Saitama 346-0101 (JP)
(74) Representative: Forstmeyer, Dietmar
(86) International application number: PCT/JP2022/002585
(87) International publication number: WO 2022/168665

(57) **Abstract**

An objective of the invention is to provide a material capable of obtaining a curable resin composition having an excellent balance between curability and storage stability. The invention is a compound represented by formula (1). 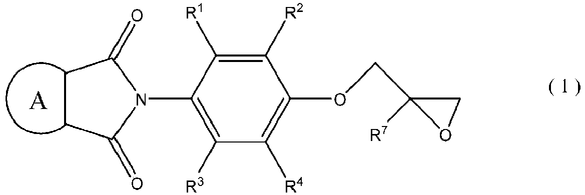 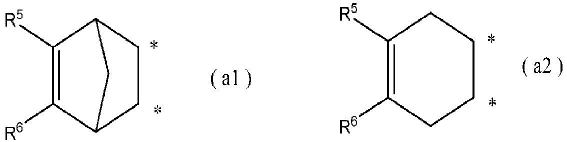 In the formula, R¹ to R⁴ each independently represent a hydrogen atom, an alkyl group having 1 to 10 carbon atoms, an alkoxy group having 1 to 10 carbon atoms, a halogen atom, a hydroxy group, a nitro group, or a nitrile group; R⁵ to R⁷ each independently represent a hydrogen atom or a methyl group; and ring A represents (a1) or (a2) and forms a fused ring with the imide ring at *.

## Description

### Technical Field

The present invention relates to a novel compound and a curable resin composition. More specifically, the present invention relates to a novel imide compound having an epoxy group, and to a curable resin composition containing an epoxy resin, a curing agent, and the aforementioned compound.

### Background Art

Epoxy resins have a wide variety of industrial uses as components for coating materials, adhesives, various molding materials, etc.

When employed for such uses, epoxy resin is typically used in combination with a curing agent. Various curing agents are known in the art, with examples including acid anhydride-based curing agents, amine-based curing agents, phenol-based curing agents, etc.

Different curing agents are used for different uses. For example, an imidazole-based curing agent, which is different from an addition polymerization-type curing agent, is an anionic polymerization-type curing agent and can therefore achieve curing even with a small additive amount, and is also useful in terms that it has low volatility and low toxicity and can therefore be suitably used for electric/electronic components.

Using an epoxy-resin curing agent alone, however, poses difficulty in striking a balance between curability and storage stability. To improve storage stability, for example, Patent Literature 1 proposes the use, in an epoxy resin system, of a reaction product between an imidazole compound and an epoxy resin. Patent Literature 2 proposes an epoxy-resin curing agent composition containing a modified imidazole, a modified amine, and a phenol compound.

Unfortunately, performing such modifications causes an increase in viscosity of the modified products, thus impairing handleability and workability and making the product unsatisfactory.

### Citation List

### Patent Literature

Patent Literature 1: US Patent No. 4066625
Patent Literature 2: JP 2007-297493A

### Summary of Invention

An objective to be achieved by the present invention is to provide a material capable of obtaining a curable resin composition having an excellent balance between curability and storage stability.

As a result of diligent research, Inventors have found that a specific compound having an imide moiety and an epoxy moiety is capable of achieving a balance between curability and storage stability of a curable resin composition, thus arriving at the present invention.

That is, the present invention is a compound represented by formula (1) below. 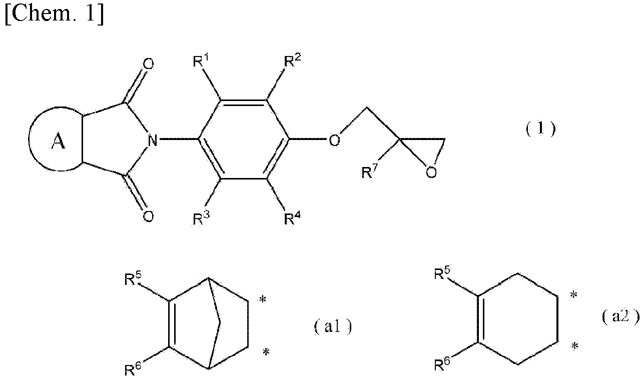 wherein, R¹ to R⁴ each independently represent a hydrogen atom, an alkyl group having 1 to 10 carbon atoms, an alkoxy group having 1 to 10 carbon atoms, a halogen atom, a hydroxy group, a nitro group, or a nitrile group; R⁵ to R⁷ each independently represent a hydrogen atom or a methyl group; and ring A represents (a1) or (a2) and forms a fused ring with the imide ring at *.

The present invention is also a curable resin composition containing an epoxy resin as component (A), a curing agent as component (B), and the aforementioned compound as component (C).

According to the present invention, it is possible to provide a curable resin composition having excellent curability and excellent storage stability. The curable resin composition of the present invention can suitably be used as a one-pack curing-type curable resin composition.

### Brief Description of Drawings

[Fig. 1] Fig. 1 is a diagram showing GPC measurement results of a compound produced in Example 1.
[Fig. 2] Fig. 2 is a diagram showing ¹H-NMR measurement results of the compound produced in Example 1.

### Description of Embodiments

Compounds according to the present invention will be described below.

A compound according to the present invention is represented by formula (1) below.

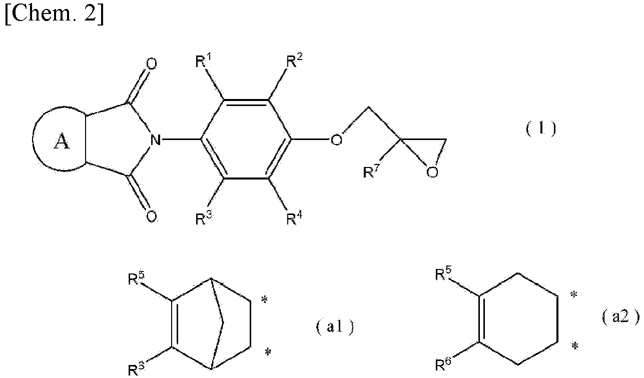

In the formula, R¹ to R⁴ each independently represent a hydrogen atom, an alkyl group having 1 to 10 carbon atoms, an alkoxy group having 1 to 10 carbon atoms, a halogen atom, a hydroxy group, a nitro group, or a nitrile group; R⁵ to R⁷ each independently represent a hydrogen atom or a methyl group; and ring A represents (a1) or (a2) and forms a fused ring with the imide ring at *.

Examples of the alkyl group having 1 to 10 carbon atoms as represented by R¹ to R⁴ in the formula (1) may include a methyl group, an ethyl group, a propyl group, an isopropyl group, a butyl group, an isobutyl group, a tert-butyl group, an amyl group, an isoamyl group, a tert-amyl group, a hexyl group, a heptyl group, an octyl group, a 2-ethylhexyl group, a nonyl group, an isononyl group, a decyl group, an isodecyl group, etc. Examples of the alkoxy group having 1 to 10 carbon atoms may include a methoxy group, an ethoxy group, a propyloxy group, an isopropyloxy group, a butoxy group, an isobutoxy group, a pentyloxy group, a hexyloxy group, a heptyloxy group, an octoxy group, a nonyloxy group, a decyloxy group, etc. Examples of the halogen atom may include fluorine, chlorine, bromine, iodine, etc.

Among compounds represented by the formula (1), compound (1A) below, wherein the ring A is (a1) and R¹ to R⁴ are each a hydrogen atom or a methyl group, is preferable from the viewpoint that a curable resin composition having an excellent balance between storage stability and curability can be obtained.

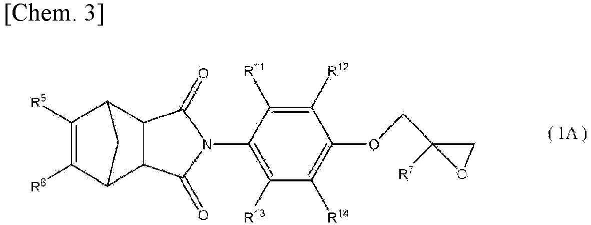

In the formula, R⁵ to R⁷ and R¹¹ to R¹⁴ each independently represent a hydrogen atom or a methyl group.

Concrete examples of particularly preferable compounds are shown below.

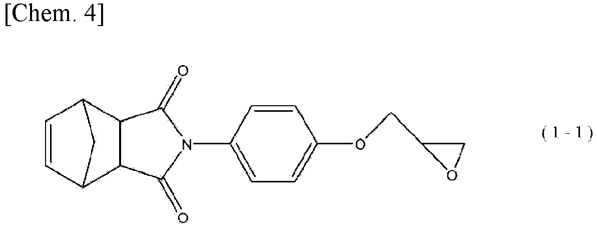

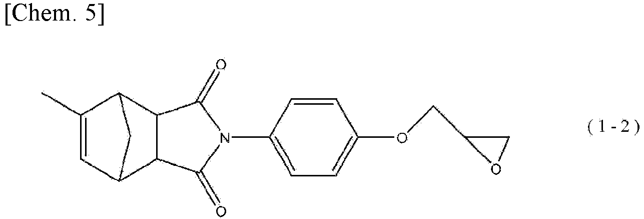

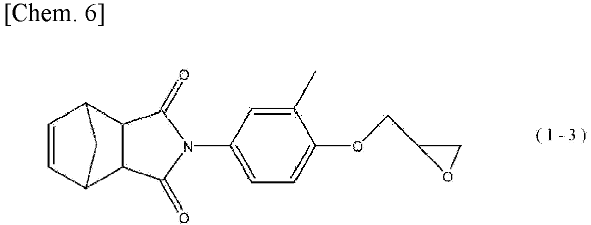

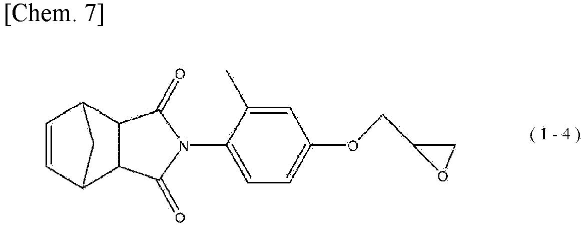

For example, the compound of the present invention can be produced according to the following scheme, although not particularly limited thereto.

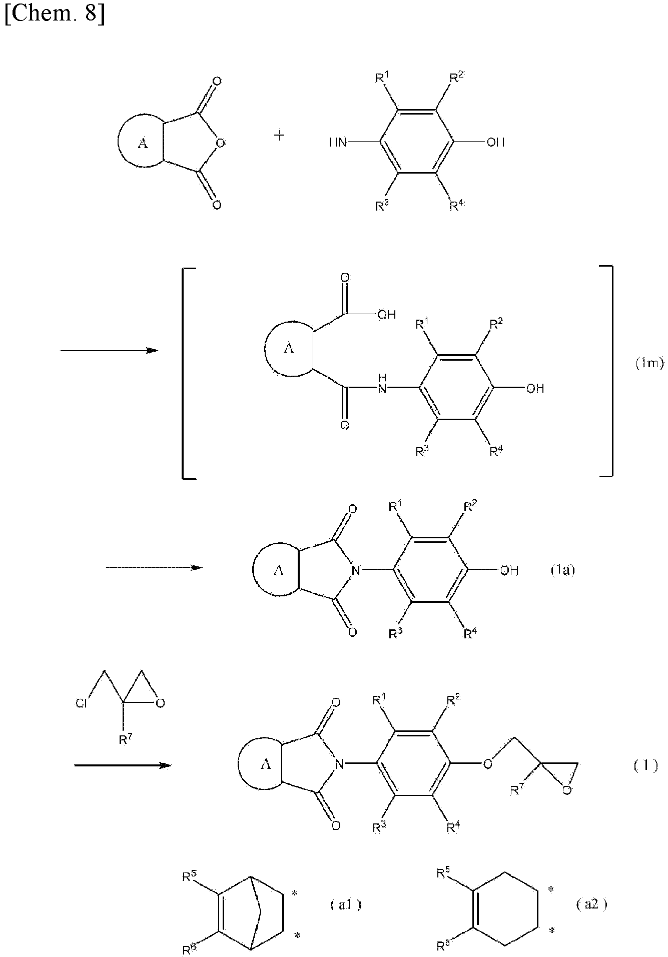

The symbols in the formulas are the same as the symbols in the formula (1) above.

A phenol compound represented by the formula (1a) can be produced by carrying out an ordinary imidization reaction using an acid anhydride having ring A and an aminophenol-based compound. Examples of methods for carrying out an imidization reaction may include the following methods.
(1) A method of synthesizing an amic acid compound (1m) by carrying out a reaction between an acid anhydride having ring A and an aminophenol-based compound under a low temperature condition of 150°C or lower, and more specifically from 0 to 120°C, preferably from 40 to 100°C, and then carrying out an imidization reaction of the amic acid compound by raising the temperature to 100 to 200°C (thermal imidization).
(2) A method of synthesizing an amic acid compound (1m) in the same manner as in method (1) above, and then carrying out an imidization reaction of the amic acid compound chemically using an imidization agent such as acetic anhydride etc. (chemical imidization).
(3) A method of synthesizing an amic acid compound in the same manner as in method (1) above, and then carrying out an imidization reaction of the amic acid compound by conducting heating under reflux in an azeotropic dehydration solvent in the presence or absence of a catalyst (azeotropic dehydration ring-closure method).

Among the methods above, method (1) is preferable.

It is preferable to carry out the imidization reaction in an organic solvent. The organic solvent to be used is not particularly limited, and examples may include: saturated hydrocarbons, such as pentane, hexane, heptane, cyclohexane, etc.; aromatic hydrocarbons, such as benzene, toluene, xylene, ethylbenzene, etc.; halogenated hydrocarbons, such as dichloromethane, chloroform, carbon tetrachloride, dichloroethane, chlorobenzene, dichlorobenzene, etc.; ethers, such as diethyl ether, diisopropyl ether, 1,2-dimethoxyethane, bis(2-methoxyethyl) ether, 1,2-bis(2-methoxyethoxy)ethane, bis[2-(2-methoxyethoxy)ethyl] ether, tetrahydrofuran, 1,3-dioxane, 1,4-dioxane, anisole, etc.; phenols, such as phenol, o-chlorophenol, m-chlorophenol, p-chlorophenol, o-cresol, m-cresol, p-cresol, 2,3-xylenol, 2,4-xylenol, 2,5-xylenol, 2,6-xylenol, 3,4-xylenol, 3,5-xylenol, etc.; amides, such as N,N-dimethylformamide, N,N-dimethylacetamide, N,N-diethylacetamide, N,N-dimethylmethoxyacetamide, hexamethylphosphoramide, etc.; lactams, such as N-methyl-2-pyrrolidone, N-methylcaprolactam, etc.; sulfur-containing solvents, such as dimethylsulfoxide, diphenylsulfoxide, dimethylsulfone, diphenylsulfone, sulfolane, etc.; and 1,3-dimethyl-2-imidazolidinone, etc. The organic solvent may be used alone, or two or more types may be used as a mixture.

The amount of organic solvent to be used is not particularly limited, but is preferably from 1 to 10000 parts by mass, more preferably from 10 to 500 parts by mass, with respect to 1 part by mass in total of the acid anhydride having ring A and the aminophenol-based compound. It is preferable to carry out the imidization reaction as a solution by dissolving the materials in an organic solvent, but the reaction may be carried out in a slurry state.

The imidization reaction may be carried out in the presence of an organic base catalyst or an acid catalyst.

Examples of the organic base catalyst may include triethylamine, tributylamine, tripentylamine, N,N-dimethylaniline, N,N-diethylaniline, pyridine, α-picoline, β-picoline, γ-picoline, 2,4-lutidine, 2,6-lutidine, quinoline, isoquinoline, etc., wherein pyridine and γ-picoline are preferable. The organic base catalyst may be used alone, or two or more types may be used in combination.

Examples of the acid catalyst may include: inorganic acids, such as hydrochloric acid, hydrogen bromide, hydrogen iodide, sulfuric acid, sulfuric anhydride, nitric acid, phosphoric acid, phosphorous acid, phosphotungstic acid, phosphomolybdic acid, etc.; sulfonic acids, such as methanesulfonic acid, ethanesulfonic acid, trifluoromethanesulfonic acid, benzenesulfonic acid, p-toluenesulfonic acid, etc.; carboxylic acids, such as acetic acid, oxalic acid, etc.; halogenated carboxylic acids, such as chloroacetic acid, dichloroacetic acid, trichloroacetic acid, fluoroacetic acid, difluoroacetic acid, trifluoroacetic acid, etc.; solid acids, such as silica, alumina, activated clay, etc.; and cationic ion-exchange resins, etc. Particularly, sulfuric acid, phosphoric acid, and p-toluenesulfonic acid are preferable. The acid catalyst may be used alone, or two or more types may be used in combination. These acid catalysts may be a salt with a diamine compound.

The amount of catalyst to be used is not particularly limited so long as the reaction rate is substantially increased, but is preferably from 0.001 to 10 mol, more preferably from 0.005 to 5 mol, even more preferably from 0.01 to 1 mol, with respect to 1 mol in total of the acid anhydride having ring A and the aminophenol-based compound.

The total reaction time of the reaction for obtaining an amic acid compound and the imidization reaction varies depending on, for example, the type of materials to be used, the type of organic solvent, the type of catalyst, the type and amount of azeotropic dehydration solvent, reaction temperature, etc., but is, for the sake of reference, from 1 to 24 hours and typically a few hours. In cases of directly carrying out thermal imidization, for the sake of reference, the reaction is carried out until the amount of distilled-out water substantially reaches the stoichiometric amount.

The reaction pressure for the reaction for obtaining an amic acid compound and the imidization reaction is not particularly limited, and may typically be at atmospheric pressure. The reaction atmosphere is not particularly limited, and may typically be in air or under an atmosphere of nitrogen, helium, neon, or argon, wherein an atmosphere of nitrogen or argon, which are inert gases, is preferable.

Methods for isolating the phenol compound represented by the formula (1a) from the reaction mixture of the acid anhydride having ring A and the aminophenol-based compound are not particularly limited. In cases where the target product has precipitated out from the reaction solvent, it may be isolated by filtration or centrifugal separation. On the other hand, in cases where the target product is still dissolved in the reaction solvent, the product may be precipitated out by, for example, removing the solvent by distillation under reduced pressure, adding an appropriate poor solvent into the reaction mixture, or discharging the reaction mixture into a poor solvent, and then be isolated by filtration or centrifugal separation.

In cases where it is necessary to further purify the isolated phenol compound represented by the formula (1a), purification may be carried out by adopting a method ordinarily known in the art, with examples including distillation purification, recrystallization, column chromatography, sludge treatment, activated carbon treatment, etc.

An example of a method for producing a compound represented by formula (1), which is the target product, from the phenol compound represented by the formula (1a) may include an ordinary glycidyl etherification reaction using epichlorohydrin or methylepichlorohydrin (referred to hereinbelow collectively as "epichlorohydrin").

A glycidyl etherification reaction is carried out by reacting the phenol compound as represented by the formula (1a) with an excessive amount of epichlorohydrin in the presence of a base, and also in the presence of a catalyst as necessary. The reaction is typically carried out at a reaction temperature of from 50 to 80°C under reduced pressure conditions of from 30 to 250 Torr. The reaction time is typically from 2 to 30 hours.

Examples of bases usable for the glycidyl etherification reaction may include sodium hydroxide, potassium hydroxide, calcium hydroxide, etc.

Examples of catalysts usable for the glycidyl etherification reaction may include Lewis acids, phase transfer catalysts, etc.

Examples of the Lewis acids may include boron trifluoride, tin chloride, zinc chloride, etc.

Examples of the phase transfer catalysts may include tetramethylammonium chloride, tetrabutylammonium bromide, methyltrioctylammonium chloride, methyltridecylammonium chloride, N,N-dimethylpyrrolidinium chloride, N-ethyl-N-methylpyrrolidinium iodide, N-butyl-N-methylpyrrolidinium bromide, N-benzyl-N-methylpyrrolidinium chloride, N-ethyl-N-methylpyrrolidinium bromide, N-butyl-N-methylmorpholinium bromide, N-butyl-N-methylmorpholinium iodide, N-allyl-N-methylmorpholinium bromide, N-methyl-N-benzyl piperidinium chloride, N-methyl-N-benzyl piperidinium bromide, N,N-dimethylpiperidinium iodide, N-methyl-N-ethylpiperidinium acetate, N-methyl-N-ethylpiperidinium iodide, etc., wherein tetramethylammonium chloride is preferable.

The amount of epichlorohydrin to be used in the glycidyl etherification reaction is preferably 1 mol or greater, particularly from 2 to 10 mol, with respect to 1 mol of the hydroxy group in the phenol compound represented by the formula (1a). The amount of base to be used is preferably from 0.1 to 2.0 mol, particularly preferably from 0.3 to 1.5 mol, with respect to 1 mol of the hydroxy group in the phenol compound represented by the formula (1a). In cases of using a phase transfer catalyst, it is preferable to use from 0.01 to 10 mass%, particularly preferably from 0.2 to 2 mass%, with respect to the phenol compound represented by the formula (1a).

The glycidyl etherification reaction may be carried out using an organic solvent, such as a hydrocarbon, an ether, a ketone, etc., but an excessive amount of epichlorohydrin may be used as a solvent.

After termination of the glycidyl etherification reaction, non-reacted epichlorohydrin is removed, for example, by distillation under reduced pressure, and the residue is dissolved in an organic solvent, such as a ketone etc., and desalted by washing with purified water, to thereby obtain a compound represented by the formula (1) as the target product.

Next, curable resin compositions according to the present invention will be described.

A curable resin composition according to the present invention contains an epoxy resin as component (A), a curing agent as component (B), and a compound represented by the formula (1) as component (C).

The epoxy resin as the component (A) that can be used is not particularly limited in terms of molecular structure, molecular weight, etc., so long as it includes at least two epoxy groups in the molecule.

Examples of the epoxy resin may include: polyglycidyl etherified products of mononuclear polyhydric phenol compounds, such as hydroquinone, resorcin, pyrocatechol, phloroglucinol, etc.; polyglycidyl etherified products of polynuclear polyhydric phenol compounds, such as dihydroxynaphthalene, biphenol, methylene bisphenol (bisphenol F), methylene bis(o-cresol), ethylidene bisphenol, isopropylidene bisphenol (bisphenol A), isopropylidene bis(o-cresol), tetrabromobisphenol A, 1,3-bis(4-hydroxycumylbenzene), 1,4-bis(4-hydroxycumylbenzene), 1,1,3-tris(4-hydroxyphenyl)butane, 1,1,2,2-tetra(4-hydroxyphenyl)ethane, thiobisphenol, sulfobisphenol, oxybisphenol, phenol novolac, o-cresol novolac, ethylphenol novolac, butylphenol novolac, octylphenol novolac, resorcin novolac, terpene phenol, etc.; polyglycidyl etherified products of polyol compounds, such as ethylene glycol, propylene glycol, butylene glycol, hexanediol, polyethylene glycol, polypropylene glycol, thioglycol, dicyclopentadiene dimethanol, 2,2-bis(4-hydroxycyclohexyl)propane (hydrogenated bisphenol A), glycerin, trimethylol propane, pentaerythritol, sorbitol, bisphenol A-alkylene oxide adduct, etc.; glycidyl esterified products of aliphatic, aromatic or alicyclic polybasic acids, such as maleic acid, fumaric acid, itaconic acid, succinic acid, glutaric acid, suberic acid, adipic acid, azelaic acid, sebacic acid, dimer acid, trimer acid, phthalic acid, isophthalic acid, terephthalic acid, trimellitic acid, trimesic acid, pyromellitic acid, tetrahydrophthalic acid, endomethylene tetrahydrophthalic acid, etc.; homopolymers or copolymers of glycidyl methacrylate; epoxy compounds containing a glycidylamino group, such as N,N-diglycidylaniline, bis(4-(N-methyl-N-glycidylamino)phenyl)methane, diglycidyl o-toluidine, N,N-bis(2,3-epoxypropyl)-4-(2,3-epoxypropoxy)-2-methylaniline, N,N-bis(2,3-epoxypropyl)-4-(2,3-epoxypropoxy)aniline, N,N,N',N'-tetra(2,3-epoxypropyl)-4,4-diaminodiphenylmethane, etc.; epoxidized products of cyclic olefin compounds, such as vinylcyclohexene diepoxide, cyclopentadiene diepoxide, 3,4-epoxycyclohexylmethyl-3,4-epoxycyclohexane carboxylate, 3,4-epoxy-6-methylcyclohexylmethyl-6-methylcyclohexane carboxylate, bis(3,4-epoxy-6-methylcyclohexylmethyl)adipate, etc.; epoxidized conjugated diene polymers, such as epoxidized polybutadiene, epoxidized styrene-butadiene copolymer, etc.; and heterocyclic compounds, such as triglycidyl isocyanurate, etc. These epoxy resins may be internally crosslinked by isocyanate-terminal prepolymers, or highly polymerized by using polyvalent active hydrogen compounds (polyhydric phenol, polyamines, carbonyl group-containing compounds, polyphosphoric esters, etc.). The epoxy resin may be used alone, or two or more types may be used in combination.

Examples of the curing agent, as the component (B), may include acid anhydride-based curing agents, phenol-based curing agents, amine-based curing agents, polythiol-based curing agents, imidazole-based curing agents, etc.

Examples of the acid anhydride-based curing agents may include hymic anhydride, phthalic anhydride, maleic anhydride, methylhymic anhydride, succinic anhydride, tetrahydrophthalic anhydride, hexahydrophthalic anhydride, methyltetrahydrophthalic anhydride, methylhexahydrophthalic anhydride, trialkyltetrahydrophthalic anhydride-maleic anhydride adducts, benzophenonetetracarboxylic anhydride, trimellitic anhydride, pyromellitic anhydride, hydrogenated methylnadic anhydride, etc.

Examples of the phenol-based curing agents may include polyhydric phenol compounds, such as phenol novolac resin, cresol novolac resin, aromatic hydrocarbon formaldehyde resin-modified phenolic resin, dicyclopentadiene-phenol addition-type resin, phenol aralkyl resin (Xylok resin), naphthol aralkyl resin, trisphenylol methane resin, tetraphenylol ethane resin, naphthol novolac resin, naphthol-phenol co-condensed novolac resin, naphthol-cresol co-condensed novolac resin, biphenyl-modified phenolic resin (a polyhydric phenol compound wherein phenol nuclei are linked by a bismethylene group), biphenyl-modified naphthol resin (a polyhydric naphthol compound wherein phenol nuclei are linked by a bismethylene group), aminotriazine-modified phenolic resin (a compound containing a phenol skeleton, a triazine ring and a primary amino group in its molecular structure), and alkoxy group-containing aromatic ring-modified novolac resin (a polyhydric phenol compound wherein a phenol nucleus and an alkoxy group-containing aromatic ring are linked by formaldehyde).

Examples of the amine-based curing agents may include: alkylene diamines, such as ethylenediamine, 1,2-diaminopropane, 1,3-diaminopropane, 1,3-diaminobutane, 1,4-diaminobutane, hexamethylenediamine, meta-xylenediamine, etc.; polyalkylpolyamines, such as diethylenetriamine, triethylenetriamine, tetraethylenepentamine, etc.; alicyclic polyamines, such as 1,4-diaminocyclohexane, 1,3-diaminocyclohexane, 1,3-diaminomethylcyclohexane, 1,2-diaminocyclohexane, 1,4-diamino-3,6-diethylcyclohexane, 4,4'-diaminodicyclohexylmethane, 1,3-bis(aminomethyl)cyclohexane, 1,4-bis(aminomethyl)cyclohexane, 4,4'-diaminodicyclohexylpropane, bis(4-aminocyclohexyl)sulfone, 4,4'-diaminodicyclohexyl ether, 2,2'-dimethyl-4,4'-diaminodicyclohexylmethane, isophorone diamine, norbornene diamine, etc.; aromatic polyamines, such as diaminodiphenylmethane, diaminodiphenylsulfone, diethyltoluenediamine, 1-methyl-3,5-diethyl-2,4-diaminobenzene, 1-methyl-3,5-diethyl-2,6-diaminobenzene, 1,3,5-triethyl-2,6-diaminobenzene, 3,3'-diethyl-4,4'-diaminodiphenylmethane, 3,5,3',5'-tetramethyl-4,4'-diaminodiphenylmethane, etc.; N,N-dimethylaminoethylamine, N,N-diethylaminoethylamine, N,N-diisopropylaminoethylamine, N,N-diallylaminoethylamine, N,N-benzylmethylaminoethylamine, N,N-dibenzylaminoethylamine, N,N-cyclohexylmethylaminoethylamine, N,N-dicyclohexylaminoethylamine, N-(2-aminoethyl)pyrrolidine, N-(2-aminoethyl)piperidine, N-(2-aminoethyl)morpholine, N-(2-aminoethyl)piperazine, N-(2-aminoethyl)-N'-methylpiperazine, N,N-dimethylaminopropylamine, N,N-diethylaminopropylamine, N,N-diisopropylaminopropylamine, N,N-diallylaminopropylamine, N,N-benzylmethylaminopropylamine, N,N-dibenzylaminopropylamine, N,N-cyclohexylmethylaminopropylamine, N,N-dicyclohexylaminopropylamine, N-(3-aminopropyl)pyrrolidine, N-(3-aminopropyl)piperidine, N-(3-aminopropyl)morpholine, N-(3-aminopropyl)piperazine, N-(3-aminopropyl)-N'-methylpiperidine, 4-(N,N-dimethylamino)benzylamine, 4-(N,N-diethylamino)benzylamine, 4-(N,N-diisopropylamino)benzylamine, N,N-dimethylisophoronediamine, N,N-dimethylbisaminocyclohexane, N,N,N'-trimethylethylenediamine, N'-ethyl-N,N-dimethylethylenediamine, N,N,N'-triethylethylenediamine, N'-ethyl-N,N-dimethylpropanediamine, N'-ethyl-N,N-dibenzylaminopropylamine; N,N-(bisaminopropyl)-N-methylamine, N,N-bisaminopropylethylamine, N,N-bisaminopropylpropylamine, N,N-bisaminopropylbutylamine, N,N-bisaminopropylpentylamine, N,N-bisaminopropylhexylamine, N,N-bisaminopropyl-2-ethylhexylamine, N,N-bisaminopropylcyclohexylamine, N,N-bisaminopropylbenzylamine, N,N-bisaminopropylallylamine, bis[3-(N,N-dimethylaminopropyl)]amine, bis[3-(N,N-diethylaminopropyl)]amine, bis[3-(N,N-diisopropylaminopropyl)]amine, bis[3-(N,N-dibutylaminopropyl)]amine, etc.; dibasic acid dihydrazides, such as oxalic acid dihydrazide, malonic acid dihydrazide, succinic acid dihydrazide, glutaric acid dihydrazide, adipic acid dihydrazide, suberic acid dihydrazide, azelaic acid dihydrazide, sebacic acid dihydrazide, phthalic acid dihydrazide, etc.; guanidine compounds, such as dicyandiamide, benzoguanamine, acetoguanamine, etc.; and melamine, etc.

It is also possible to use modified amine-based curing agents obtained by modifying the aforementioned amines. Examples of modification methods may include dehydration condensation with carboxylic acids, addition reaction with epoxy resins, addition reaction with isocyanates, Michael addition reaction, Mannich reaction, condensation reaction with urea, and condensation reaction with ketones.

Examples of carboxylic acids that can be used for modification of the aforementioned amines may include aliphatic, aromatic or alicyclic polybasic acids such as maleic acid, fumaric acid, itaconic acid, succinic acid, glutaric acid, suberic acid, adipic acid, azelaic acid, sebacic acid, dimer acid, trimer acid, phthalic acid, isophthalic acid, terephthalic acid, trimellitic acid, trimesic acid, pyromellitic acid, tetrahydrophthalic acid, hexahydrophthalic acid, endomethylene tetrahydrophthalic acid, etc.

Examples of epoxy compounds that can be used for modification of the aforementioned amines may include the epoxy compounds given as examples for the aforementioned epoxy resin which is the component (A).

Examples of isocyanate compounds that can be used for modification of the aforementioned amines may include: aromatic diisocyanates, such as 2,4-tolylene diisocyanate, 2,6-tolylene diisocyanate, diphenylmethane-4,4'-diisocyanate, phenylene diisocyanate, xylylene diisocyanate, tetramethylxylylene diisocyanate, 1,5-naphthylene diisocyanate, 1,5-tetrahydronaphthalene diisocyanate, 3,3'-dimethyldiphenyl-4,4'-diisocyanate, dianisidine diisocyanate, tetramethylxylylene diisocyanate, etc.; alicyclic diisocyanates, such as isophorone diisocyanate, dicyclohexylmethane-4,4'-diisocyanate, trans-1,4-cyclohexyl diisocyanate, norbornene diisocyanate, etc.; aliphatic diisocyanates, such as tetramethylene diisocyanate, 1,6-hexamethylene diisocyanate, 2,2,4- and/or 2,4,4-trimethylhexamethylene diisocyanate, lycine diisocyanate, etc.; isocyanurate trimers, biuret trimers, trimethylolpropane adducts, etc., of the aforementioned diisocyanates; triphenylmethane triisocyanate, 1-methylbenzol-2,4,6-triisocyanate, dimethyltriphenylmethane tetraisocyanate, etc. Further, the aforementioned isocyanate compounds may take the form of a modified product, such as carbodiimide-modified, isocyanurate-modified, biuret-modified, etc., or may take the form of blocked isocyanate which is blocked by a variety of blocking agents.

Examples of the polythiol-based curing agents may include pentaerythritol tetrakis(3-mercaptopropionate), pentaerythritol tetrakis(thioglycolate), dipentaerythritol hexakis(3-mercaptopropionate), dipentaerythritol hexakis(3-mercaptobutyrate), 1,3,4,6-tetrakis(2-mercaptoethyl)-1,3,4,6-tetraazaoctahydropentalene-2,5-dione, 1,3,5-tris(3-mercaptopropyl)-1,3,5-triazine-2,4,6(1H,3H,5H)-trione, 4-mercaptomethyl-1,8-dimercapto-3,6-dithiaoctane, 4,8-, 4,7- or 5,7-dimercaptomethyl-1, 1 1-dimercapto-3,6,9-trithiaundecane, 1,3,4,6-tetrakis(2-mercaptoethyl)glycoluril, etc.

Examples of the imidazole-based curing agents may include 2-methylimidazole, 2-ethyl-4-methylimidazole, 2-isopropylimidazole, 2-undecylimidazole, 2-heptadecylimidazole, 2-phenylimidazole, 2-phenyl-4-methylimidazole, 2-aminopropylimidazole, 1-phenylmethyl-2-imidazole, 1-phenylmethyl-2-ethyl-4-methylimidazole, 1-phenylmethyl-2-phenylimidazole, 1-butoxycarbonylethyl-2-methylimidazole, 1-butoxycarbonylethyl-2-ethyl-4-methylimidazole, 1-butoxycarbonylethyl-2-phenylimidazole, 1-(2-ethylhexyl)carbonylethyl-2-methylimidazole, 1-(2-ethylhexyl)carbonylethyl-2-ethyl-4-methylimidazole, 1-(2-ethylhexyl)carbonylethyl-2-phenylimidazole, 1-octyloxycarbonylethyl-2-methylimidazole, 1-octyloxycarbonylethyl-2-ethyl-4-methylimidazole, 1-octyloxycarbonylethyl-2-phenylimidazole, hexanediol·bis(2-methylimidazolyl ethanoic acid) ester, hexanediol·bis(2-ethyl-4-methylimidazolyl ethanoic acid) ester, hexanediol·bis(2-phenylimidazolyl ethanoic acid) ester, decanediol·bis(2-methylimidazolyl ethanoic acid) ester, decanediol·bis(2-ethyl-4-methylimidazolyl ethanoic acid) ester, decanediol·bis(2-phenylimidazolyl ethanoic acid) ester, tricyclopentane dimethanol·bis(2-methylimidazolyl ethanoic acid) ester, tricyclopentane dimethanol·bis(2-ethyl-4-methylimidazolyl ethanoic acid) ester, tricyclopentane dimethanol·bis(2-phenylimidazolyl ethanoic acid) ester, 1-(2-hydroxynaphthylmethyl)-2-methylimidazole, 1-(2-hydroxynaphthylmethyl)-2-ethyl-4-methylimidazole, 1-(2-hydroxynaphthylmethyl)-2-phenylimidazole, imidazole silane (for example, 2MUSIZ from Shikoku Chemicals Corporation), etc. The above may be used in the form of salts with trimellitic acid, isocyanuric acid, boron, etc. Further, it is also possible to use modified imidazole-based curing agents obtained by modifying these imidazole compounds in the same manner as the aforementioned modified amine-based curing agents.

Examples of commercially available curing agents usable as the component (B) may include: Adeka Hardener EH-3636AS and Adeka Hardener EH-4351S (from Adeka Corporation; dicyandiamide-type latent curing agent); Adeka Hardener EH-5011S and Adeka Hardener EH-5046S (from Adeka Corporation; imidazole-type latent curing agent); Adeka Hardener EH-4357S, Adeka Hardener EH-5057P, and Adeka Hardener EH-5057PK (from Adeka Corporation; polyamine-type latent curing agent); Ajicure PN-23 and Ajicure PN-40 (from Ajinomoto Fine-Techno Co., Inc.; amine adduct-based latent curing agent); Ajicure VDH (from Ajinomoto Fine-Techno Co., Inc.; hydrazide-based latent curing agent); Fujicure FXR-1020 (from T&K TOKA Corporation; latent curing agent); Curezol (from Shikoku Chemicals Corporation; imidazole-based curing agent); TS-G (from Shikoku Chemicals Corporation; polythiol-based curing agent); DPMP and PEMP (from SC Organic Chemical Co., Ltd.; polythiol-based curing agent); and PETG (from Yodo Kagaku Co., Ltd.; polythiol-based curing agent), etc.

The curing agent may be used alone, or two or more types may be used in combination.

In the present invention, it is preferable that the curing agent is an imidazole-based curing agent, more preferably an unmodified compound, such as 2-methylimidazole, 2-ethyl-4-methylimidazole, etc., because curing is possible with a relatively small usage amount, and it is also possible to achieve effects as a curing accelerator when used in combination with another curing agent.

The amount of curing agent to be added is not particularly limited, but is preferably from 1 to 70 parts by mass, more preferably from 1 to 50 parts by mass, even more preferably from 3 to 30 parts by mass, with respect to 100 parts by mass of the epoxy resin which is the component (A).

In the present invention, it is possible to use, in combination, the aforementioned curing agent and a known epoxy-resin curing accelerator, as necessary. Examples of the curing accelerator may include: phosphines, such as triphenylphosphine, etc.; phosphonium salts, such as tetraphenylphosphonium bromide, etc.; amines, such as benzyldimethylamine, 2,4,6-tris(dimethylaminomethyl)phenol, etc.; quaternary ammonium salts, such as trimethylammonium chloride, etc.; ureas, such as 3-(p-chlorophenyl)-1, 1-dimethylurea, 3-(3,4-dichlorophenyl)-1,1-dimethylurea, 3-phenyl-1,1-dimethylurea, isophorone diisocyanate-dimethylurea, tolylene diisocyanate-dimethylurea, etc.; and complexes between boron trifluoride and amines, complexes between boron trifluoride and ether compounds, etc. The curing accelerator may be used alone, or two or more types may be used in combination. The content of the epoxy-resin curing accelerator is not particularly limited, and may be set as appropriate depending on the use of the curable resin composition.

The curable resin composition according to the present invention contains a compound represented by the aforementioned formula (1) as the component (C).

Particularly, it is preferable to contain at least one type of compound represented by the aforementioned formulas (1-1) to (1-4) as the component (C), in terms that a curable resin composition having excellent curability and storage stability can be obtained.

The content of the component (C) with respect to 100 parts by mass of the component (B) is preferably from 1 to 2000 parts by mass, more preferably from 10 to 1000 parts by mass, even more preferably from 30 to 500 parts by mass. If the content of the component (C) is less than 1 part by mass, it may not be possible to achieve the curable resin composition's effect of imparting stability. If the content exceeds 2000 parts by mass, curability may be negatively affected.

The curable resin composition of the present invention may contain an antioxidant, with examples including phosphorus-based antioxidants, phenol-based antioxidants, sulfur-based antioxidants, etc.

Examples of the phosphorus-based antioxidants may include triphenyl phosphite, tris(2,4-di-tert-butylphenyl) phosphite, tris(nonylphenyl) phosphite, tris(dinonylphenyl) phosphite, tris(mono- and di-nonylphenyl) phosphite, bis(2-tert-butyl-4,6-dimethylphenyl)·ethyl phosphite, diphenyl acid phosphite, 2,2'-methylene-bis(4,6-di-tert-butylphenyl)octyl phosphite, diphenyl decyl phosphite, phenyl diisodecyl phosphite, tributyl phosphite, tris(2-ethylhexyl) phosphite, tridecyl phosphite, trilauryl phosphite, dibutyl acid phosphite, dilauryl acid phosphite, trilauryl trithio phosphite, bis(neopentyl glycol) . 1,4-cyclohexane dimethyl diphosphite, bis(2,4-di-tert-butylphenyl) pentaerythritol diphosphite, bis(2,6-di-tert-butyl-4-methylphenyl) pentaerythritol diphosphite, distearyl pentaerythritol diphosphite, phenyl-4,4'-isopropylidene diphenol pentaerythritol diphosphite, tetra(C12 to C15 mixed alkyl)-4,4'-isopropylidene diphenyl phosphite, bis[2,2'-methylene-bis(4,6-diamylphenyl)]·isopropylidene diphenyl phosphite, hydrogenated 4,4'-isopropylidene diphenol polyphosphite, bis(octylphenyl)·bis[4,4'-n-butylidene-bis(2-tert-butyl-5-methylphenol)]·1,6-hexanediol diphosphite, tetratridecyl-4,4'-butylidene-bis(2-tert-butyl-5-methylphenol) diphosphite, hexa(tridecyl)·1,1,3-tris(2-methyl-5-tert-butyl-4-hydroxyphenyl)butane-triphosphonite, 9,10-dihydro-9-oxa-10-phosphaphenanthrene-10-oxide, 2-butyl-2-ethylpropanediol·2,4,6-tri-tert-butylphenol monophosphite, etc.

Examples of the phenol-based antioxidants may include 2,6-di-tert-butyl-p-cresol, 2,6-diphenyl-4-octadecyloxyphenol, stearyl(3,5-di-tert-butyl-4-hydroxyphenyl)propionate, distearyl(3,5-di-tert-butyl-4-hydroxybenzyl)phosphonate, tridecyl-3,5-di-tert-butyl-4-hydroxybenzyl thioacetate, thiodiethylene-bis[(3,5-di-tert-butyl-4-hydroxyphenyl)propionate], 4,4'-thiobis(6-tert-butyl-m-cresol), 2-octylthio-4,6-di(3,5-di-tert-butyl-4-hydroxyphenoxy)-s-triazine, 2,2'-methylene-bis(4-methyl-6-tert-butylphenol), bis[3,3-bis(4-hydroxy-3-tert-butylphenyl)butyric acid] glycol ester, 4,4'-butylidene-bis(4,6-di-tert-butylphenol), 2,2'-ethylidene-bis(4,6-di-tert-butylphenol), 1,1,3-tris(2-methyl-4-hydroxy-5-tert-butylphenyl)butane, bis[2-tert-butyl-4-methyl-6-(2-hydroxy-3-tert-butyl-5-methylbenzyl)phenyl]terephthalate, 1,3,5-tris(2,6-dimethyl-3-hydroxy-4-tert-butylbenzyl)isocyanurate, 1,3, 5-tris(3, 5-di-tert-butyl-4-hydroxybenzyl)isocyanurate, 1,3, 5-tris(3, 5-di-tert-butyl-4-hydroxybenzyl)-2,4,6-trimethylbenzene, 1,3,5-tris[(3,5-di-tert-butyl-4-hydroxyphenyl)propionyloxyethyl]isocyanurate, tetrakis[methylene-3-(3,5-di-tert-butyl-4-hydroxyphenyl)propionate]methane, 2-tert-butyl-4-methyl-6-(2-acryloyloxy-3-tert-butyl-5-methylbenzyl)phenol, 3,9-bis[2-(3-tert-butyl-4-hydroxy-5-methylhydrocinnamoyloxy)-1,1-dimethylethyl]-2,4,8, 10-tetraoxaspiro[5.5]undecane, triethylene glycol bis[β-(3-tert-butyl-4-hydroxy-5-methylphenyl)propionate], etc.

Examples of the sulfur-based antioxidants may include dialkyl thiodipropionates, such as dilauryl, dimyristyl, myristylstearyl and distearyl esters of thiodipropionic acid, and β-alkylmercaptopropionate esters of polyols, such as pentaerythritol tetra(β-dodecylmercaptopropionate), etc.

The curable resin composition of the present invention may contain a UV absorber and/or a light stabilizer, such as a hindered-amine-based light stabilizer.

Examples of the UV absorber may include: 2-hydroxybenzophenones, such as 2,4-dihydroxybenzophenone, 2-hydroxy-4-methoxybenzophenone, 2-hydroxy-4-octoxybenzophenone, 2-hydroxy-4-tert-butyl-4'-(2-methacryloyloxyethoxyethoxy)benzophenone, 5,5'-methylene-bis(2-hydroxy-4-methoxybenzophenone), etc.; 2-(2-hydroxyphenyl)benzotriazoles, such as 2-(2-hydroxy-5-methylphenyl)benzotriazole, 2-(2-hydroxy-5-tert-octylphenyl)benzotriazole, 2-(2-hydroxy-3,5-di-tert-butylphenyl)-5-chlorobenzotriazole, 2-(2-hydroxy-3-tert-butyl-5-methylphenyl)-5-chlorobenzotriazole, 2-(2-hydroxy-3-dodecyl-5-methylphenyl)benzotriazole, 2-(2-hydroxy-3-tert-butyl-5-C7 to C9 mixed alkoxycarbonylethylphenyl)triazole, 2-(2-hydroxy-3,5-dicumylphenyl)benzotriazole, 2,2'-methylene-bis(4-tert-octyl-6-benzotriazolyl phenol), 2-(2-hydroxy-3-tert-butyl-5-carboxyphenyl)benzotriazole polyethylene glycol ester, etc.; 2-(2-hydroxyphenyl)-1,3,5-triazines, such as 2-(2-hydroxy-4-hexyloxyphenyl)-4,6-diphenyl-1,3,5-triazine, 2-(2-hydroxy-4-methoxyphenyl)-4,6-diphenyl-1,3,5-triazine, 2-(2-hydroxy-4-octoxyphenyl)-4,6-bis(2,4-dimethylphenyl)-1,3,5-triazine, etc.; benzoates, such as phenyl salicylate, resorcinol monobenzoate, 2,4-di-tert-butylphenyl-3,5-di-tert-butyl-4-hydroxybenzoate, 2,4-di-tert-amylphenyl-3,5-di-tert-butyl-4-hydroxybenzoate, hexadecyl-3,5-di-tert-butyl-4-hydroxybenzoate, etc.; substituted oxanilides, such as 2-ethyl-2'-ethoxyoxanilide, 2-ethoxy-4'-dodecyloxanilide, etc.; and cyanoacrylates, such as ethyl-α-cyano-β,β-diphenylacrylate, methyl-2-cyano-3-methyl-3-(p-methoxyphenyl)acrylate, etc.

Examples of the hindered-amine-based light stabilizer may include 2,2,6,6-tetramethyl-4-piperidyl stearate, 1,2,2,6,6-pentamethyl-4-piperidyl stearate, 2,2,6,6-tetramethyl-4-piperidyl benzoate, bis(2,2,6,6-tetramethyl-4-piperidyl)sebacate, bis(1,2,2,6,6-pentamethyl-4-piperidyl)sebacate, tetrakis(2,2,6,6-tetramethyl-4-piperidyl)-1,2,3,4-butane tetracarboxylate, tetrakis(1,2,2,6,6-pentamethyl-4-piperidyl)-1,2,3,4-butane tetracarboxylate, bis(2,2,6,6-tetramethyl-4-piperidyl)·bis(tridecyl)-1,2,3,4-butane tetracarboxylate, bis(1,2,2,6,6-pentamethyl-4-piperidyl)·bis(tridecyl)-1,2,3,4-butane tetracarboxylate, bis(1,2,2,6,6-pentamethyl-4-piperidyl)-2-butyl-2-(3,5-di-tert-butyl-4-hydroxybenzyl)malo nate, 1-(2-hydroxyethyl)-2,2,6,6-tetramethyl-4-piperidinol/diethyl succinate polycondensate, 1,6-bis(2,2,6,6-tetramethyl-4-piperidylamino)hexane/dibromoethane polycondensate, 1,6-bis(2,2,6,6-tetramethyl-4-piperidylamino)hexane/2,4-dichloro-6-morpholino-s-triazin e polycondensate, 1,6-bis(2,2,6,6-tetramethyl-4-piperidylamino)hexane/2,4-dichloro-6-tert-octylamino-s-tria zine polycondensate, 1,5,8,12-tetrakis[2,4-bis(N-butyl-N-(2,2,6,6-tetramethyl-4-piperidyl)amino)-s-triazin-6-yl ]-1,5,8,12-tetraazadodecane, 1,5,8,12-tetrakis[2,4-bis(N-butyl-N-(1,2,2,6,6-pentamethyl-4-piperidyl)amino)-s-triazin-6 -yl]-1, 5, 8,12-tetraazadodecane, 1,6,11-tris[2,4-bis(N-butyl-N-(2,2,6,6-tetramethyl-4-piperidyl)amino)-s-triazin-6-ylamino ]undecane, 1,6,11-tris[2,4-bis(N-butyl-N-(1,2,2,6,6-pentamethyl-4-piperidyl)amino-s-triazin-6-ylami no)]undecane, etc.

The curable resin composition of the present invention may contain a silane coupling agent.

Examples of the silane coupling agent may include γ-aminopropyltriethoxysilane, N-β-(aminoethyl)-γ-aminopropyltriethoxysilane, N-β-(aminoethyl)-N'-β-(aminoethyl)-γ-aminopropyltriethoxysilane, γ-anilinopropyltriethoxysilane, γ-glycidoxypropyltriethoxysilane, β-(3,4-epoxycyclohexyl)ethyltriethoxysilane, vinyltriethoxysilane, N-β-(N-vinylbenzylaminoethyl)-γ-aminopropyltriethoxysilane, γ-methacryloxypropyltrimethoxysilane, γ-chloropropyltrimethoxysilane, γ-mercaptopropyltrimethoxysilane, etc.

The curable resin composition of the present invention may contain a filler.

Examples of the filler may include: silica, such as fused silica, crystalline silica, etc.; powders, such as magnesium hydroxide, aluminum hydroxide, zinc molybdate, calcium carbonate, silicon carbonate, calcium silicate, potassium titanate, beryllia, zirconia, zircon, forsterite, steatite, spinel, mullite, titania, etc., and beads obtained by conglobing the above; and fibers, such as glass fiber, pulp fiber, synthetic fiber, ceramic fiber, etc.

The curable resin composition of the present invention may contain one or more of various solvents, preferably organic solvents.

Examples of the organic solvents may include: ethers, such as tetrahydrofuran, 1,2-dimethoxyethane, 1,2-diethoxyethane, etc.; alcohols, such as isobutanol, n-butanol, isopropanol, n-propanol, amyl alcohol, benzyl alcohol, furfuryl alcohol, tetrahydrofurfuryl alcohol, etc.; ketones, such as methyl ethyl ketone, methyl isopropyl ketone, methyl butyl ketone, etc.; aromatic hydrocarbons, such as benzene, toluene, xylene, etc.; triethylamine, pyridine, dioxane, acetonitrile, etc.

The curable resin composition of the present invention may further contain other types of additives, as necessary.

Examples of the additives may include: phenol compounds, such as biphenol, etc.; reactive diluents, such as monoalkyl glycidyl ether, etc.; non-reactive diluents (plasticizers), such as dioctyl phthalate, dibutyl phthalate, benzyl alcohol, coal tar, etc.; reinforcing materials, such as glass cloth, aramid cloth, carbon fiber, etc.; pigments; lubricants, such as candelilla wax, carnauba wax, Japan wax, Chinese wax, beeswax, lanolin, spermaceti wax, montan wax, petroleum wax, aliphatic wax, aliphatic ester, aliphatic ether, aromatic ester, aromatic ether, etc.; thickeners; thixotropic agents; antifoaming agents; rust preventives; and commonly used additives, such as colloidal silica, colloidal alumina, etc. In the present invention, an adhesive resin, such as cyanate ester resin, xylene resin, petroleum resin, etc., may be used in combination.

The curable resin composition of the present invention allows the balance between curability and storage stability to be adjusted, and can therefore be used as a one-pack curing-type curable resin composition. The uses of the curable resin composition of the present invention are not particularly limited, and examples may include adhesives for electronic components, sealing materials for electronic components, casting materials, coating materials, structural adhesives, etc.

### Examples

Next, the present invention will be described in further detail below according to Examples and Comparative Example. The invention, however, is not to be limited to the following Examples.

### {Example 1}

A flask equipped with a rotator, a thermometer, and a Dean-Stark apparatus was charged with 65.7 g (0.4 mol) of norbornene dicarboxylic anhydride, 43.7 g (0.4 mol) of p-aminophenol, and 350 g of xylene, and the mixture was stirred at 100°C for 2 hours. To this mixture, 3.4 g (0.02 mol) of p-toluene sulfonic acid and 50 g of N-methylpyrrolidone were added and the temperature was raised, and the mixture was subjected to dehydration under reflux for 4 hours. After cooling to room temperature, 500 mL of water was added and stirred. The solid was filtered out and recrystallized with methanol/toluene, and further, the crystal was filtered out, to obtain a purple crystal (melting point: 251°C).

A flask equipped with a rotator, a thermometer, and a Dean-Stark apparatus was charged with 25.5 g (0.1 mol) of the obtained purple crystal and 46.3 g (0.5 mol) of epichlorohydrin, and the temperature was raised until the solution refluxed, and then, 8.3 g (0.1 mol) of a 48% sodium hydroxide aqueous solution was dropped from a dropping funnel over 1 hour. The mixture was further stirred for 2 hours, and was then cooled to room temperature. The reaction solution was washed with 50 g of distilled water, and epichlorohydrin was removed by distillation under reduced pressure. Then, 100 g of toluene was added to dissolve the residue, followed by washing with 50 g of distilled water. Toluene was removed by distillation, to obtain a light-brown viscous liquid.

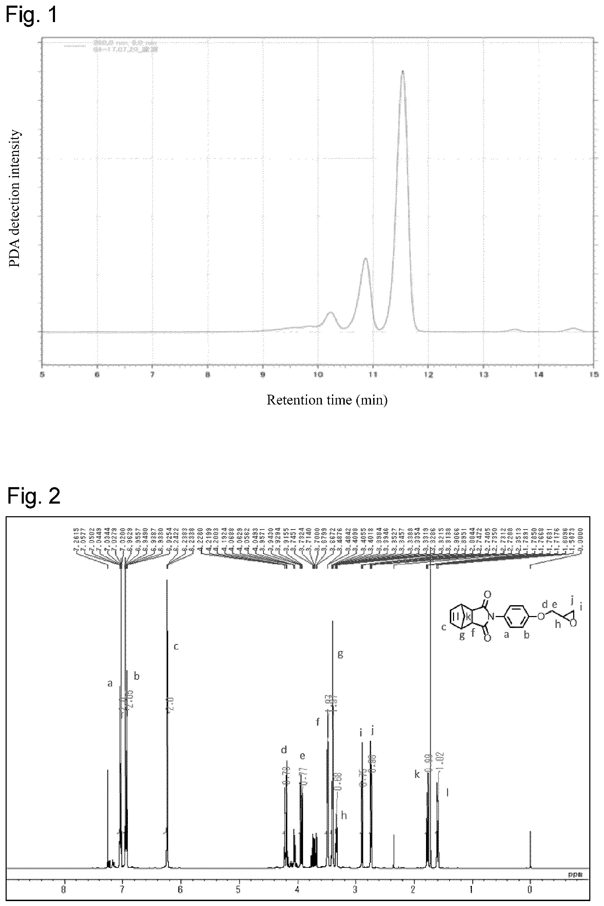

The obtained viscous liquid was left to stand for a day, resulting in a light-brown crystal with a melting point of 100°C. The crystal was used to conduct GPC measurement and ¹H-NMR measurement. The results are respectively shown in Figs. 1 and 2. The epoxy equivalent of the obtained light-brown crystal was 413 g/eq. (theoretical value: 311 g/eq.).

From these results, it was verified that the obtained light-brown crystal was a compound represented by the aforementioned formula (1-1).

### {Examples 2 to 7}

As shown in Table 1, curable resin compositions were produced, respectively using Adeka Resin EP-4100E (from Adeka Corporation; bisphenol A-type epoxy resin) as an epoxy resin of component (A), 2-ethyl-4-methylimidazole (2E4MZ) or Adeka Hardener EH-2021 (from Adeka Corporation; adduct-type imidazole) as a curing agent of component (B), and the compound obtained by Example 1 as component (C), and the compositions were evaluated in terms of storage stability and curability.

### {Comparative Example 1}

A curable resin composition was produced in the same manner as in Example 2, except that the compound obtained by Example 1 was not used, and the composition was evaluated in terms of storage stability and curability.

### Storage Stability:

Evaluation of storage stability was conducted by placing each curable resin composition in a beaker and leaving it to stand at atmospheric temperature, and counting the number of days it took for it to gelate. If the composition does not gelate within 3 days, there is no practical problem, and the composition is found as having excellent storage stability.

### Curability:

Evaluation of curability was conducted by coating a glass plate with each curable resin composition using a bar coater, and observing the curing state when heated at 150°C for 2 hours. A composition with no tack was evaluated as ∘ (white circle), a composition having tack was evaluated as Δ (white triangle), and a composition in liquid form was evaluated as × (cross). The composition is found as having excellent curability in cases of ∘ and Δ.

**[Table 1]**

| | | Example | | | | | | Comparative Example |
|---|---|---|---|---|---|---|---|---|
| | | 2 | 3 | 4 | 5 | 6 | 7 | 1 |
| (A) | EP-4100E (g) | 100 | 100 | 100 | 100 | 100 | 100 | 100 |
| (B) | 2E4MZ (g) | 5 | 5 | 5 | | | | 5 |
| | EH-2021 (g) | | | | 10 | 10 | 10 | |
| (C) | Compound of Example 1 (g) | 100 | 25 | 10 | 100 | 25 | 10 | |
| Evaluation | Storage stability (day s) | 15 | 8 | 5 | 20 | 10 | 7 | 1 |
| | Curability | Δ | ○ | ○ | Δ | ○ | ○ | ○ |

The aforementioned Example 1 obtained a novel compound, i.e., the compound represented by the aforementioned formula (1-1). Table 1 clearly shows that the curable resin compositions employing this compound in combination with an epoxy resin and a curing agent have better curability and storage stability compared to a curable resin composition that does not employ this compound.

### Industrial Applicability

According to the present invention, it is possible to provide a one-pack curing-type curable resin composition that particularly has excellent curability and storage stability, and that can suitably be used, for example, for adhesives for electronic components, sealing materials for electronic components, casting materials, coating materials, structural adhesives, etc.

## Claims

1. A compound represented by formula (1) below: 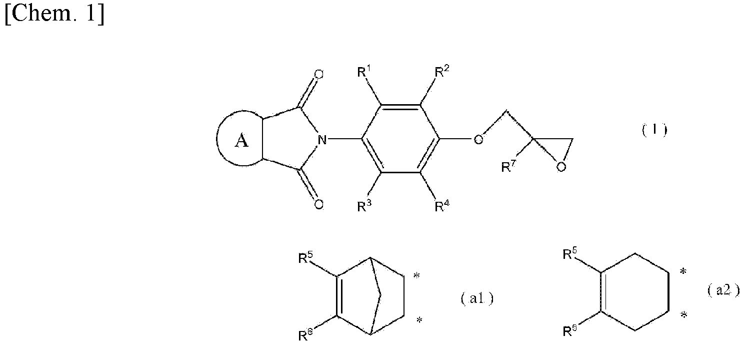
wherein, R¹ to R⁴ each independently represent a hydrogen atom, an alkyl group having 1 to 10 carbon atoms, an alkoxy group having 1 to 10 carbon atoms, a halogen atom, a hydroxy group, a nitro group, or a nitrile group;
R⁵ to R⁷ each independently represent a hydrogen atom or a methyl group; and
ring A represents (a1) or (a2) and forms a fused ring with the imide ring at *.

2. The compound according to claim 1, represented by formula (1A) below: 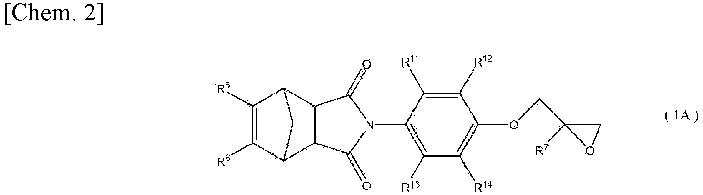 wherein, R⁵ to R⁷ and R¹¹ to R¹⁴ each independently represent a hydrogen atom or a methyl group.

3. A curable resin composition comprising an epoxy resin as component (A), a curing agent as component (B), and the compound according to claim 1 or 2 as component (C).

4. The curable resin composition according to claim 3, wherein the curing agent as the component (B) includes an imidazole-based curing agent.

5. The curable resin composition according to claim 3 or 4, wherein a content of the curing agent as the component (B) is from 0.01 to 100 parts by mass with respect to 100 parts by mass of the epoxy resin as the component (A).

6. The curable resin composition according to any one of claims 3 to 5, wherein a content of the compound as the component (C) is from 1 to 2000 parts by mass with respect to 100 parts by mass of the curing agent as the component (B).

7. A cured product of the curable resin composition according to any one of claims 3 to 6.

8. The curable resin composition according to any one of claims 3 to 6, wherein the curable resin composition is a one-pack curing-type composition.
